# EUROPÄISCHE PATENTSCHRIFT

(11) **EP 2 105 111 B1**
(45) Veröffentlichungstag und Bekanntmachung des Hinweises auf die Patenterteilung: **27.04.2011**
(21) Anmeldenummer: 09154203.5
(22) Anmeldetag: 03.03.2009
(51) Int. Cl.: A61F 2/90

(54) **Medizinisches Stützimplantat, insbesondere Stent**
Medical implant, in particular stent
Implant de soutien médical, en particulier un stent

(30) Priorität: 29.03.2008 DE 102008016363
(43) Veröffentlichungstag der Anmeldung: 30.09.2009
(73) Patentinhaber: Biotronik VI Patent AG, 6341 Baar (CH)
(72) Erfinder: Mews, Steffen, 18057, Rostock (DE); Bakczewitz, Frank, 18055, Rostock (DE)
(74) Vertreter: Lindner-Vogt, Karin L.

(56) Entgegenhaltungen:
- WO-A-99/40874
- WO-A-2006/107608
- US-A- 5 797 951
- US-A1- 2003 004 563

## Beschreibung

Die Erfindung betrifft ein medizinisches Stützimplantat und insbesondere einen Stent mit den im Oberbegriff des Anspruches 1 angegebenen Merkmalen.

Derartige medizinische Stützimplantate kommen in erster Linie zum Einsatz zur Therapie von Stenosen in Koronargefäßen, sind jedoch auch bei anderen Indikationen in andersartigen Gefäßen des Körpers verwendbar. In der Regel weisen solche Stützimplantate einen röhrenartigen Grundkörper auf, der sich aus biegbaren Streben zusammensetzt. Letztere sind aus dem Grundkörper durch Laserschneiden herausgearbeitet. Für die Strebenstruktur sind dabei unterschiedlichste Designs bekannt, wobei für den vorliegenden Fall als Ausgangspunkt für die Erfindung solche Stentdesigns mit in Peripherrichtung mäanderförmig umlaufenden ^{T}ragstegen betroffen sind, bei denen an Knotenpunkten von mindestens zwei Streben diese unter einem spitzen Winkel zueinander stehen. Beim Dilatieren des Implantats vergrößert sich dieser spitze Winkel unter einer Biegebeanspruchung der Streben bei dem Knoten.Problematisch bei derartigen Stents ist die Tatsache, dass während des Crimpens, also dem Komprimieren des Stents in seinen Zustand, in den er an einem Ballonkatheter in das jeweilige Gefäß eingeführt wird, und der anschließenden Dilatation starke plastische Verformungen auftreten, die einen entscheidenden Einfluss auf die elastische Rückfederung der Stentstruktur haben. Es ist anzustreben, dass der als Recoil bezeichnete Rückfederungseffekt möglichst gering gehalten wird. Dies kann in einem begrenzten Maß durch örtliche plastische Verformungen der Stentstruktur beispielsweise im Bereich der Knotenpunkte zwischen benachbarten Streben der umlaufenden Tragstege gewährleistet werden. Der plastischen Verformung sind allerdings materialbedingt Grenzen gesetzt, bei deren Überschreiten die Gefahr eines Strukturversagens beispielsweise bei Erreichen der Bruchdehnung im Material gegeben ist.

Durch die Einschränkung, materialspezifische Grenzwerte nicht zu überschreiten, ist es entsprechend problematisch, ein gutes Recoil-Verhalten des Stents zu erreichen. Auch ist eine Querschnittsvergrößerung der Streben der Stentstruktur nur eingeschränkt möglich. US 2003/004563 A1 zeigt einen Stent, dessen Streben an Knotenpunkten im spitzen Winkel zueinander stehen. Bei Aufweitung erfährt dieser Stent eine Längenkürzung.

Zur Losung der vorstehenden ProblemantiK sind aus dem stand der Technik bereits Ansatzpunkte bekannt, Stentstrukturen durch mechanische Konstruktionen ohne plastische Deformation expandierbar zu machen. So offenbart die US 5,824,054 A ein Gefäß-Stützimplantat, das aus einem auf sich gewickelten PTFE-Folienblatt mit umlaufenden Perforationsreihen besteht. In die Perforationen können an das Folienblatt angeformte Laschen eingreifen, die so gerichtet sind, dass ein Komprimieren des Stents verhindert wird.

Aus der US 5,441,515 A ist ein dort so bezeichneter "Ratschen-Stent" bekannt, bei dem die zylindrische Stentstruktur durch in Umfangsrichtung verlaufende Zungen gebildet ist. Diese sind über eine gemeinsame Verbindungstraverse zusammengehalten, in der jeder Zunge zugeordnet ein Schlitz für den Eingriff des Zungenendes angeordnet ist. Die Zungen sind an ihren seitlichen Rändern mit Rastzahnreihen versehen, deren Rastzähne unidirektional wirken. Die Zungenenden sind in der komprimierten Stellung tief in die Schlitze eingesteckt, beim Dilatieren des Stents ziehen sich die Zungen allmählich aus den Schlitzen nach außen, wobei eine Kontraktion danach durch die mit den Schlitzenden in Eingriff stehenden Rastzähne verhindert wird.

Die US 2007/0061004 A1 schließlich zeigt einen expandierenden Stent, bei dem in Umfangsrichtung verlaufende Gitterstrukturen in einzelne Abschnitte unterteilt sind, die ein Aufweiten der Stentstruktur durch eine Verschiebung der Einzelelemente zueinander erlauben. Dabei greifen die Gitterstrukturen mit laschenartigen Fortsätzen in entsprechende Öffnungen der benachbarten Gitterstruktur ein.

US5797951 zeigt einen Stent mit Ratschenmechanismus, bei dem Stentringe bei der Dilatation mittels eines Ratschenmechanismus aufgeweitet werden können.

Der Erfindung liegt nun die Aufgabe zugrunde, ein Stentdesign für Stützimplantate anzugeben, bei dem unter lediglich begrenzter Deformation der plastischen Verformungsbereiche ein größerer Aufweitbereich aus dem gecrimpten Zustand heraus realisierbar ist.

Diese Aufgabe wird laut Kennzeichnungsteil des Anspruchs 1 dadurch gelöst, dass zumindest ein Teil der Streben zwischen den plastischen Verformungsbereichen mit einem teleskopierbaren Ratschenmechanismus zur irreversiblen Verlängerung der jeweiligen Strebe beim Dilatieren des Stents versehen ist.

Das erfindungsgemäße Stützimplantat folgt also in seiner Grundstruktur dem konventionellen ballon-expandierbaren Design, das aus mäanderförmig umlaufenden Tragstegen besteht. Diese werden während der Dilatation in den Knotenpunkten zwischen zwei benachbarten Streben plastisch verformt. Zusätzlich werden die mäanderförmigen Tragstege in ihren unverformten, geraden Passagen zwischen ihren plastischen Verformungsbereichen mit einem Ratschenmechanismus versehen, der vorzugsweise aus zwei einander flankierenden Strebenarmen gebildet ist, die durch unidirektional wirkende Rastzähne in Verlängerungsrichtung zueinander verschiebbar, in Gegenrichtung dazu jedoch gesperrt sind. Durch die Relativbewegung der beteiligten Strebenarme kann ein über die plastische Verformung deutlich hinausgehender Durchmesserzuwachs erreicht werden. Dabei wird ein Zurückrutschen der sich flankierenden Strebenarme und somit eine Rückfederung der gesamten Struktur aufgrund der Verrastung der Stege miteinander verhindert. Der Dilatationsweg setzt sich also zum einen aus der plastischen Verformung der Mäanderschlaufen und zum anderen aus der Teleskopbewegung der verlängerbaren Streben in Umfangsrichtung zusammen. Da der elastische Recoil nur aus der Verformung der Stege selbst herrührt, wird dieser insgesamt reduziert.

Weitere Merkmale, Einzelheiten und Vorteile der Erfindung ergeben sich aus der nachfolgenden Beschreibung, in der ein Ausführungsbeispiel anhand der beigefügten Zeichnungen näher erläutert wird. Es zeigen:
- Fig. 1: eine ausschnittsweise vergrößerte Detailansicht einer Stentstruktur im ge- crimpten Zustand in abgewickelter Darstellung,
- Fig. 2: eine Detailansicht der Stentstruktur analog Fig. 1 in dilatiertem Zustand,
- Fig. 3: eine perspektivische Darstellung eines Strebenabschnitts in nicht dilatiertem Ausgangszustand, und
- Fig. 4: eine perspektivische Darstellung des Strebenabschnitts aus anderer Blickrich- tung in einem dilatierten Zustand.

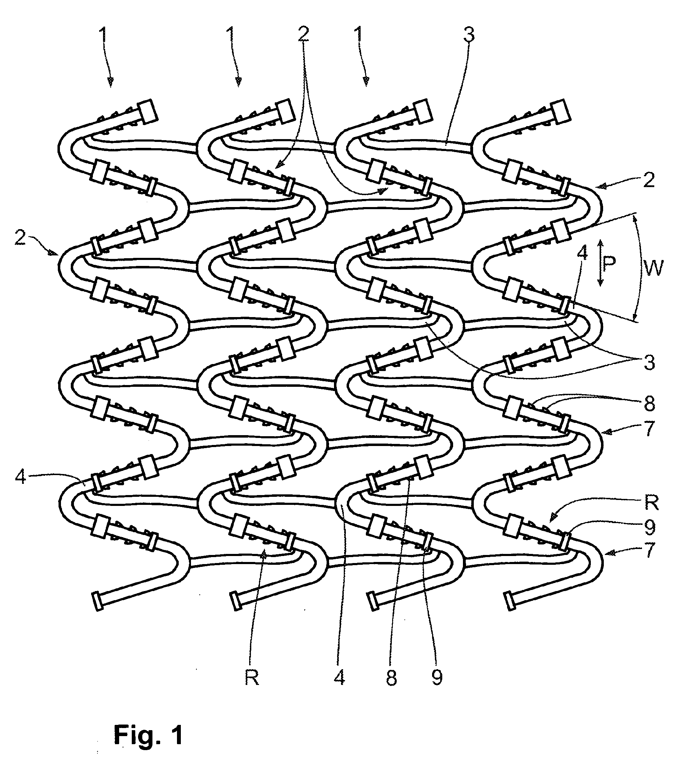

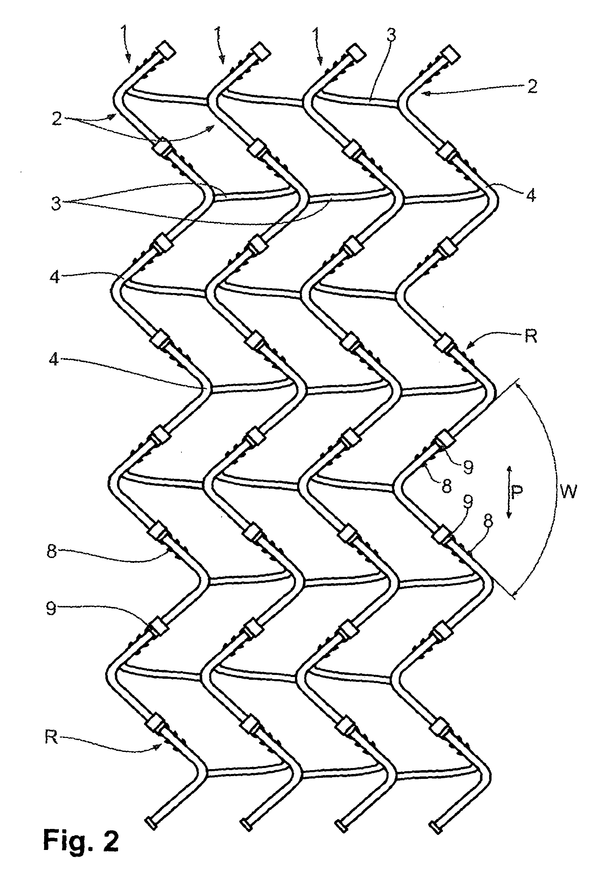

Wie aus Fig. 1 und 2 deutlich wird, besteht die Stentstruktur aus in Peripherrichtung P umlaufenden, mäanderförmigen Tragstegen 1, die sich aus in spitzem Winkel W einander anschließenden Streben 2 zusammensetzen. Parallel zur Längsrichtung des Stents 1 sind die einzelnen Tragstege durch Axialverbinder 3 miteinander gekoppelt. Insgesamt wird aus der in Fig. 1 gezeigten Stentstruktur also ein (nicht dargestellter) röhrenartiger Grundkörper gebildet, der aus einer gecrimpten, kontrahierten Grundstellung durch radiale Ausdehnung mithilfe beispielsweise eines Ballons in eine Stützstellung in einem Gefäß dilatierbar ist. Beim Dilatationsvorgang werden dabei in erster Linie die Knotenpunkte 4 zwischen zwei benachbarten Streben 2 aufgedehnt und entsprechend plastisch verformt. Dadurch vergrößert sich der Winkel W unter Biegebeanspruchung der Streben 2 im jeweiligen Knotenpunkt 4, wie dies aus Fig. 2 deutlich wird.

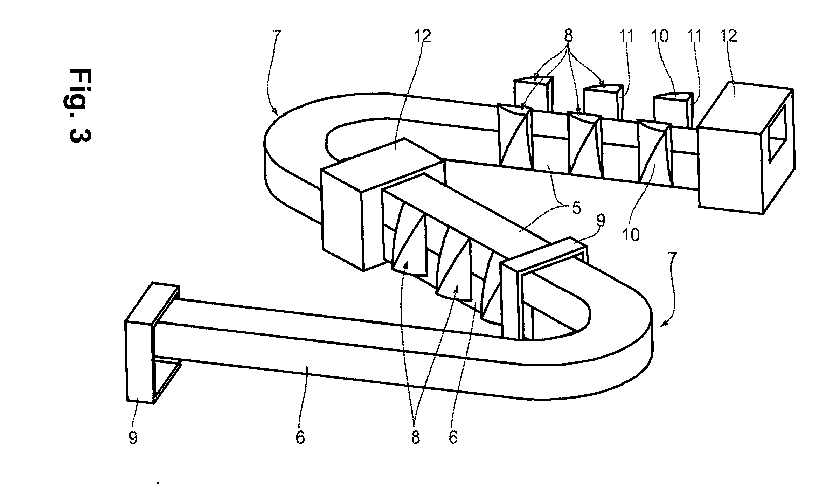

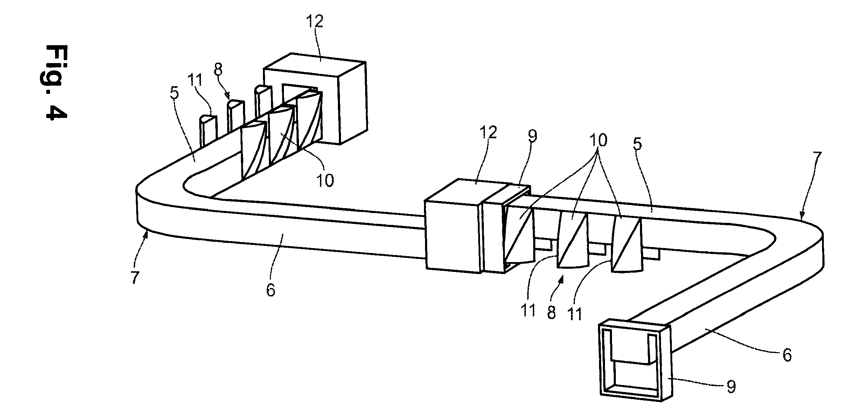

Die Streben 2 sind - wie aus Fig. 3 und 4 deutlich wird - jeweils durch zwei einander flankierende Strebenarme 5, 6 gebildet. Jeder Tragsteg 1 setzt sich also aus etwa V-förmigen Strebenkomponenten 7 zusammen, deren Schenkel die Strebenarme 5, 6 bilden. Seitlich an den Strebenarmen 5 sind jeweils unidirektional wirkende Rastzähne 8 angeformt, die gemeinsam mit einer rahmenförmigen, quer zur Armlängsrichtung angeordneten Rastmanschette 9 am freien Ende des anderen Strebenarmes 6 einen Ratschenmechanismus R bilden, mit dessen Hilfe jede Strebe 2 beim Dilatieren irreversibel verlängert werden kann. Wie aus Fig. 3 in Verbindung mit Fig. 1 deutlich wird, sind in der kontrahierten Ausgangsstellung des Stents die beiden Strebenarme 5, 6 einer Strebe 2 jeweils zumindest zu einem großen Längenanteil aufeinander zu geschoben, ergeben also eine kleine Länge der Strebe 2. Beim Dilatieren des Stents werden durch die entsprechende Zugbelastung die beiden Strebenarme 5, 6 auseinander gezogen, wobei die Rastzähne 8 sukzessive mit ihrer Gleitflanke 10 unter der Rastmanschette 9 durchschnappen können. Aufgrund der steilen Sperrflanke 11 jedes Rastzahns 8 sind dann die beiden Strebenarme 5, 6 gegen ein Zusammenschieben nach dem Dilatieren aufgrund der Radialbelastung des Stents im Gefäß gesichert.

Wie in den Fig. 3 und 4 erkennbar ist, ist die Gleitflanke 10 der Rastzähne 8 in zwei orthogonalen Ebenen, nämlich in Längsrichtung der Strebenarmes 5 und quer dazu abgerundet gestaltet, sodass die Rastmanschetten 9 leichter darüber hinweg gleiten können.

Die Strebenarme 5 mit den Rastzähnen 8 sind ferner an ihrem freien Ende mit einem blockartigen Anschlag 12 versehen, der mit der Rastmanschette 9 des anderen Strebenarmes 6 kooperiert. Durch diese Längenbegrenzung der relativen Teleskopverschiebung der beiden Strebenarme 5, 6 wird - wie der Fig. 4 entnehmbar ist - verhindert, dass sich die Verbindung zwischen den beiden Armen 5, 6 löst.

Insgesamt lässt sich der Ratschenmechanismus R mit einem Kabelbinder-Rastmechanismus vergleichen. Der Stent weist - generell gesprochen - ein Recoil-Verhalten nur aufgrund der plastischen Verformung der Streben 2 auf, das aufgrund der erreichten Verlängerung der Streben 2 mit Hilfe des teleskopierbaren Ratschenmechanismus R und der damit verbundenen Durchmesservergrößerung des Stents ohne Erhöhung der Biegebelastung geringer ausfällt als bei vergleichbaren Stentstrukturen ohne diesen Ratschenmechanismus R.

## Patentansprüche

1. Medizinisches Stützimplantat, insbesondere Stent, umfassend einen röhrenartigen Grundkörper, der aus biegbaren Streben (2) zusammengesetzt ist, wobei an Knotenpunkten (4) von mindestens zwei Streben (2) diese unter einem spitzen Winkel (W) zueinander stehen, der sich beim Dilatieren des Implantats unter plastischer Verformung der Streben (2) bei dem Knotenpunkt (4) vergrößert,
**dadurch gekennzeichnet, dass** zumindest ein Teil der Streben (2) zwischen den plastischen Verformungsbereichen (4) mit einem teleskopierbaren Ratschenmechanismus (R) zur irreversiblen Verlängerung der jeweiligen Strebe (2) beim Dilatieren versehen ist.

2. Medizinisches Stützimplantat nach Anspruch 1, **dadurch gekennzeichnet, dass** der Ratschenmechanismus (R) jeweils durch zwei einander flankierende Strebenarme (5, 6) gebildet ist, die durch unidirektional wirkende Rastzähne (8) in Verlängerungsrichtung zueinander verschiebbar, in Gegenrichtung dazu gesperrt sind.

3. Medizinisches Stützimplantat nach Anspruch 2, **dadurch gekennzeichnet, dass** mehrere Rastzähne (8) an einem Strebenarm (5, 6) aneinander gereiht angeordnet sind.

4. Medizinisches Stützimplantat nach Anspruch 2 oder 3, **dadurch gekennzeichnet, dass** die Rastzähne (8) eines Strebenarmes (6) jeweils mit einer Rastmanschette (9) am flankierenden Strebenarm (5) nach Art eines Kabelbinder-Rastmechanismus kooperieren.

5. Medizinisches Stützimplantat nach einem der vorgenannten Ansprüche, **dadurch gekennzeichnet, dass** die Rastzähne (8) jeweils mit einer steilen Sperrflanke (11) und einer Gleitflanke (10) versehen sind.

6. Medizinisches Stützimplantat nach Anspruch 5, **dadurch gekennzeichnet, dass** die Gleitflanke (10) der Rastzähne (8) in zwei im Wesentlichen orthogonalen Ebenen abgerundet geformt ist.

7. Medizinisches Stützimplantat nach einem der vorgenannten Ansprüche, **dadurch gekennzeichnet, dass** die mit den Rastzähnen (8) versehenen Strebenarme (5) an ihren Enden Anschläge (12) zur Längenbegrenzung der relativen Teleskopbewegung der Strebenarme (5, 6) zueinander aufweisen.

## Claims

1. A medical supporting implant, in particular stent, comprising a tubular base body composed of flexible struts (2), wherein at junction points (4) of at least two struts (2), the latter are situated relative to one another at an acute angle (W) which enlarges when the implant is dilated, thereby plastically deforming the struts (2),
**characterized in that** at least one portion of the struts (2) between the plastic deformation regions (4) is equipped with a telescopic ratcheting mechanism (R) for the irreversible extension of the particular strut (2) upon dilation.

2. The medical supporting implant according to claim 1, **characterized in that** each ratcheting mechanism (R) is formed by two mutually flanking strut arms (5, 6) which can be displaced relative to one another in the lengthening direction by way of unidirectionally acting locking teeth (8), but which are blocked in the opposite direction.

3. The medical supporting implant according to claim 2, **characterized in that** a plurality of locking teeth (8) are disposed on a strut arm (5, 6) in a row.

4. The medical supporting implant according to claim 2 or 3, **characterized in that** the locking teeth (8) on a strut arm (6) cooperate in each case with a locking cuff (9) on the flanking strut arm (5) in the manner of a cable-tie locking mechanism.

5. The medical supporting implant according to one of the preceding claims, **characterized in that** each of the locking teeth (8) is equipped with a steep locking flank (11) and a gliding flank (10).

6. The medical supporting implant according to claim 5, **characterized in that** the gliding flank (10) of the locking teeth (8) is rounded in shape in two substantially orthogonal planes.

7. The medical supporting implant according to one of the preceding claims, **characterized in that** the strut arms (5) equipped with the locking teeth (8) comprise stops (12) on the ends thereof for limiting the length of the relative telescopic motion of the strut arms (5, 6) relative to one another.

## Revendications

1. Implant de support médical, en particulier un stent, comprenant un corps de base du genre tubulaire, composé de tiges flexibles (2), dans lequel, aux points de croisement (4) d'au moins deux tiges (2), celles-ci forment un angle aigu (W) qui s'agrandit lors de la dilatation de l'implant sous déformation plastique des tiges (2) au point de croisement (4),
**caractérisé en ce qu'**au moins une partie des tiges (2) entre les régions de déformation plastique (4) est pourvue d'un mécanisme à rochet télescopique (R), pour l'extension irréversible de chacune des tiges (2) lors de la dilatation.

2. Implant de support médical selon la revendication 1, **caractérisé en ce que** le mécanisme à rochet (R) est formé respectivement par deux bras de tige d'encadrement (5, 6), déplaçables l'un par rapport à l'autre dans la direction d'extension, par des dents d'arrêt (8) agissant de façon unidirectionnelle, et verrouillés dans la direction contraire.

3. Implant de support médical selon la revendication 2, **caractérisé en ce que** plusieurs dents d'arrêt (8) sont juxtaposées sur un bras de tige (5, 6).

4. Implant de support médical selon la revendication 2 ou 3, **caractérisé en ce que** les dents d'arrêt (8) d'un bras de tige (6) coopèrent respectivement avec une manchette d'arrêt (9) sur le bras de tige d'encadrement (5), à la manière d'un mécanisme à rochet d'attache-câbles.

5. Implant de support médical selon l'une des revendications précédentes, **caractérisé en ce que** les dents d'arrêt (8) sont respectivement pourvues d'un flanc de blocage abrupt (11) et d'un flanc de glissement (10).

6. Implant de support médical selon la revendication 5, **caractérisé en ce que** le flanc de glissement (10) des dents d'arrêt (8) est formé de façon arrondie sur deux plans essentiellement orthogonaux.

7. Implant de support médical selon l'une des revendications précédentes, **caractérisé en ce que** les bras de tige (5) pourvus de dents d'arrêt (8) comportent, à leurs extrémités, des butées (12) pour la délimitation longitudinale du mouvement télescopique relatif des bras de tige (5, 6) les uns par rapport aux autres.
